# EUROPÄISCHE PATENTSCHRIFT

(11) **EP 3 426 200 B1**
(45) Veröffentlichungstag und Bekanntmachung des Hinweises auf die Patenterteilung: **22.01.2020**
(21) Anmeldenummer: 17709065.1
(22) Anmeldetag: 07.03.2017
(51) Int. Cl.: A61F 5/01, A61F 5/02

(54) **FLEXIBLES STÜTZELEMENT FÜR EINE ORTHESE**
FLEXIBLE SUPPORT FOR AN ORTHOSIS
SUPPORT FLEXIBLE POUR UNE ORTHÈSE

(30) Priorität: 08.03.2016 DE 102016203780
(43) Veröffentlichungstag der Anmeldung: 16.01.2019
(73) Patentinhaber: Bauerfeind AG, 07937 Zeulenroda-Triebes (DE)
(72) Erfinder: SCHÜTZE, Frank, 07937 Langenwolschendorf (DE); BAUERFEIND, Hans B., 07937 Zeulenroda-Triebes (DE)
(74) Vertreter: Schwahn, Hartmut
(86) Internationale Anmeldenummer: PCT/EP2017/055242
(87) Internationale Veröffentlichungsnummer: WO 2017/153364

(56) Entgegenhaltungen:
- EP-A2- 2 923 686
- US-A- 4 144 881
- US-A- 4 573 455
- US-A1- 2006 211 967

## Beschreibung

Die Erfindung betrifft ein flexibles Stützelement für eine Orthese aus einer Gliederkette mit mehreren gleichartigen Kettengliedern, die miteinander überlappend über jeweils einen gemeinsamen Lagerzapfen zugfest verkoppelt und dabei begrenzt gegeneinander verschwenkbar sind, um eine Flexibilität des Stützelements zu erreichen.

Orthesen dienen als medizinische Hilfsmittel der Stabilisierung, Entlastung, Ruhigstellung und insbesondere auch der Führung oder Korrektur von Gliedmaßen sowie von Schulter, Becken und Wirbelsäule eines Patienten. Die mechanische Stabilisierung und die Führung oder Korrektur wird dabei besonders erreicht durch mechanisch steife Stabilisierungselemente in der Orthese, die über geeignete Bandagen und/oder Gurtelemente mit dem Körper in festen mechanischen Kontakt gebracht werden, so dass Stützkräfte aufgenommen oder Korrekturkräfte ausgeübt werden können. Es sind sogenannte Hartrahmenorthesen für Extremitätengelenke, beispielsweise Kniegelenksorthesen bekannt, worin Gelenkschienen, besonders beiderseits des Gelenks, das Gelenk überbrücken und so mit der Extremität mechanisch fest verbunden sind, um Stützkräfte aufzunehmen und die Bewegung des Gelenks zu stützen und/oder zu korrigieren. Die Gelenkschienen sind dazu über geeignete Mittel wie Bandagen und Gurte distal und proximal des Gelenks mit der Extremität fest verbunden; im Falle einer Kniegelenksorthese über sogenannte Unterschenkel- und Oberschenkelfassungen. Dabei sind die beiderseitigen Gelenkschienen über sogenannte "Brücken" mechanisch fest miteinander verbunden, um so den starren Hartrahmen der Orthese zu bilden. Vergleichbare Konstruktionen sind für Ellenbogen-, Handgelenk- und Fußgelenksorthesen bekannt.

Nachteilig bei bekannten Hartrahmenorthesen, besonders Kniegelenksorthesen, sind gerade diese starren Brücken zwischen den beiden seitlichen Gelenkschienen. Diese müssen zum Einen mechanisch fest ausgebildet sein, um die beiden Gelenkschienen sinnvoll miteinander zu verkoppeln. Bekannte Konstruktionen können daher aber nicht genau genug an die Kontur der Extremität angepasst werden, mit welcher sie mechanisch fest verbunden werden müssen. Es zeigt sich jedoch, dass sich, abhängig von der Bewegungsphase und der Belastung der Extremität, vor allem wegen der damit einhergehenden Muskelverformung, die äußere Form und Umfang der Extremität lokal verändert. Es besteht damit das Problem, dass ein solcher Hartrahmen bei Bewegung nicht mehr ausreichend gehalten werden kann und aus der gewünschten Position verrutscht, das heißt "migriert", und so die Stützwirkung, aber auch der Tragekomfort signifikant beeinträchtigt werden kann. Bisherige Lösungen dieses Problems schlagen dazu festere mechanische Verspannungen oder Vergurtungen zwischen Hartrahmen und Extremität vor. Dies soll dann durch zusätzliche Gurte, die fest gespannt werden und/oder durch rutschhemmende Beschichtungen auf der zur Extremität gerichteten Seite des Hartrahmens erreicht werden. Dadurch werden aber der Tragekomfort und damit die Akzeptanz des Patienten für eine Hartrahmenorthese verschlechtert. Dies führt zum Beispiel dazu, dass der Patient die therapeutisch zweckmäßige Gelenkbewegung nicht mehr vollständig durchführt und eine Schonhaltung einnimmt, welche einer Therapie entgegenstehen kann. Es ist wünschenswert, eine Hartrahmenorthese bereitzustellen, welche in jeder Bewegungsphase fest und sicher am Körper des Patienten anliegt.

Die US 4573455 A und die US 4144881 A offenbaren ein flexibles Stützband für eine Orthese mit einer flachen Gliederkette aus mehreren gleichartigen sich überlappenden Gliedern als Gelenkschiene für Gelenk- und Rückenorthesen.

Ein anderer Nachteil solcher Hartrahmen mit starren Kopplungsbrücken zwischen den Gelenkschienen ist, dass sie an verschiedene Körpergrößen, das heißt besonders Umfänge der Extremität, nicht ohne weiteres anpassbar sind. Insbesondere auch im Verlauf der Therapie, beispielsweise bei einer muskelaufbauenden Rehabilitation, kann sich der Umfang der Extremität ändern; ein starrer Hartrahmen passt dann nicht mehr. Es ist ein Hartrahmen für eine stützende oder korrigierende Orthese wünschenswert, welcher flexibel an den jeweiligen Umfang der Extremität angepasst werden kann.

Rückenorthesen zur Stützung und/oder Korrektur von Becken und/oder Wirbelsäule enthalten in der Regel starre seitlich oder vor allem zentral über der Wirbelsäule verlaufende Stützelemente oder Stützschienen. Diese werden insbesondere durch Gurte oder Bandagen gegen Becken und/oder Wirbelsäule gepresst, um durch entsprechende Krafteinleitung eine Stütz- oder Korrekturwirkung auf Becken und/oder Wirbelsäule zu entfalten. Nachteilig bei bekannten Rückenorthesen oder Wirbelsäulenorthesen ist, dass solche starren Stützelemente der Bewegung des Patienten nur unzureichend folgen können. Zwar soll ja eine mechanische Stütz- und Korrekturwirkung erzielt werden, weshalb diese Stützelemente mechanisch fest sein und Kräfte aufnehmen können müssen. Es zeigt sich aber, dass gerade in der gezielt geführten Bewegung ein höherer Therapieerfolg liegen kann, als in einem ganz starren Rahmen, welcher wegen seiner mechanischen Festigkeit keine wesentliche Bewegung erlaubt. Besonders auch bei Patienten mit Skoliose sind herkömmliche "gerade" Stützschienen, welche lediglich der Lordose und/oder Kyphose der Wirbelsäule folgen, nicht ausreichend an eine zusätzliche seitliche Verkrümmung der Wirbelsäule anpassbar. Es sind daher Becken- und Wirbelsäulenorthesen wünschenswert, worin mechanisch feste Stützelemente, welche die Stützwirkung vermitteln, so ausgebildet sind, dass sie der Bewegung des Patienten teilweise oder weitgehend folgen können, trotzdem aber mechanische Stützwirkung besitzen und ungewünschte Bewegungen begrenzen können.

Der vorliegenden Erfindung lag daher das technische Problem zugrunde, an sich mechanisch starre Stützelemente von Orthesen so weiterzubilden, dass sie eine gewisse Flexibilität und Anpassbarkeit ermöglichen, gleichzeitig aber mechanisch derart fest sind und Kräfte aufnehmen können, um die gewünschte mechanische Stützwirkung ausüben zu können. Ein Aspekt ist dabei, dass diese in an sich bekannten Orthesenkonstruktionen herkömmliche starre Stütz- oder Koppelelemente ersetzen können.

Das technische Problem wird vollständig gelöst durch die Bereitstellung eines flexiblen Stützbands für eine Orthese nach Anspruch 1, welches aus einer flachen Gliederkette aufgebaut ist, worin mehrere gleichartige flache Glieder miteinander verbunden sind. Dabei ist jedes Glied mit dem jeweils benachbarten Glied räumlichkörperlich überlappend und zugfest verkoppelt. Die Glieder sind dabei besonders aus einem mechanisch festen Werkstoff, besonders einem Kunststoff, Metall oder einem Materialverbund (z.B. Sandwich oder Inserts) gefertigt, sind an sich also mechanisch weitgehend starr.

Zur zugfesten Verkopplung dient hierbei jeweils ein Koppel- oder Lagerzapfen. Dieser ist besonders zu den flachen Gliedern im Wesentlichen senkrecht ausgerichtet. Die jeweils verkoppelten Glieder sind in ihrer gemeinsamen Achse dieses Lagerzapfens gegeneinander verschwenkbar. Erfindungsgemäß übergreifen sich die jeweils verbundenen Glieder der Kette, das heißt sie überlappen sich gegenseitig in der Fläche. An den überlappenden Abschnitten der Glieder sind erfindungsgemäß Schultern ausgebildet, an denen das jeweils benachbarte Glied körperlich anschlägt, wodurch die jeweilige Verschwenkung der Glieder zueinander in der Achse des Lagerzapfens begrenzt ist.

Auf diese Weise ist ein neuartiges teil-flexibles Stützelement in Form eines flachen Bandes bereitgestellt, welches ausreichend mechanisch fest ist, insbesondere in seiner Längsausrichtung zug- und stauchfest ist, jedoch aus seiner Längsausrichtung aus einer geraden Erstreckung heraus zu einem gewissen Grad seitwärts biegbar und damit flexibel ist. Diese Biegbarkeit und Flexibilität wird durch die Verschwenkbarkeit der Glieder der Kette zueinander in der Achse des sie jeweils verkoppelnden Lagerzapfens ermöglicht. Diese Verschwenkung, und damit die Biegung und Flexibilität in Längsrichtung der Kette, wird aber durch die erfindungsgemäße gegenseitige räumliche Hemmung der Glieder an Schultern der jeweils benachbarten Glieder limitiert.

Unter einem Stützband wird hier ein grundsätzlich flaches, band- oder schienenförmiges Konstrukt verstanden, das als Bestandteil einer Orthese oder eines festen Orthesenrahmens als festes Stützelement oder als ein weitere Stützelemente oder Gelenkelemente verbindendes Brückenelement dienen kann.
Ein erster Gegenstand der Erfindung ist daher ein solches flexibles Stützband für eine Orthese, welches eine flache Gliederkette aus mehreren, insbesondere gleichartigen flachen Gliedern enthält, wobei jeweils ein erstes Glied mit einem benachbarten zweiten Glied an jeweils untergreifenden und übergreifenden Gliederabschnitten körperlich überlappend zueinander angeordnet und über einen dazu im Wesentlichen senkrechten Zapfen miteinander zugfest verkoppelt sind, wobei die Glieder um die Achse dieses gemeinsamen Zapfens gegeneinander verschwenkbar sind. An einem überlappenden (untergreifenden und übergreifenden) Gliederabschnitt ist eine Schulter gebildet, die einen Endanschlag zur Limitierung dieser Verschwenkung bildet.
In einer bevorzugten Ausgestaltung ist der Lagerzapfen an dem untergreifenden einen Gliederabschnitt des ersten Gliedes ausgebildet und eine entsprechende Lagerbuchse oder Ausnehmung zur Aufnahme des Lagerzapfens an dem übergreifenden anderen Gliederabschnitt des benachbarten zweiten Glieds ausgebildet. Der Lagerzapfen greift in dieser Ausgestaltung in die Lagerbuchse ein, um die Glieder zugfest zu verkoppeln.
In einer besonderen Ausgestaltung weist der Lagerzapfen einen gegenüber dem Durchmesser der Lagerbuchse, worin der Lagerzapfen einrasten soll, verdickten Kopf aus, welcher ein formschlüssiges Einrasten des Lagerzapfens in die Lagerbuchse des benachbarten Glieds ermöglicht und ein Herausgleiten des Lagerzapfens aus der Lagerbuchse gegen einen Widerstand verhindert.
Erfindungsgemäß weisen die Kettenglieder zur Verhinderung des Ausrutschens des Lagers und der Entkopplung der Glieder voneinander zusätzliche Rastnasen auf, die in entsprechenden Ausnehmungen des benachbarten Glieds eingreifen, um ein Abheben des einen Gliedes von dem benachbarten Kettenglied räumlich zu verhindern.

In einer anderen Ausgestaltung ist der Lagerzapfen als Schraube oder Niet ausgebildet. In einer spezifischen Ausgestaltung davon sind in beiden zu verkoppelnden Gliedern jeweils Ausnehmungen in Form von Lagerbuchsen vorgesehen, wobei die Glieder jeweils durch einen separaten Niet oder Bolzen, der durch beide Lagerbuchsen getrieben ist, oder eine separate Schraube, die dort eingeschraubt ist, verkoppelt sind. Niet oder Schraube sind in an sich bekannter Weise gesichert: der Niet durch insbesondere beiderseitige Nietköpfe, die Schraube insbesondere durch Schraubenkopf und eine als Gewindebohrung ausgebildete Lagerbuchse.

Bevorzugt sind Lagerzapfen und Lagerbuchse in allen Ausgestaltungen jeweils mittig in Bezug auf die Längsausrichtung der Gliederkette, das heißt auf der Mittellinie positioniert; bevorzugt sind Lagerzapfen und Lagerbuchse außermittig (exzentrisch) zu der Querachse jedes Gliedes an dem Glied positioniert.

Besonders ist vorgesehen, dass zumindest eine der Schultern, die den Endanschlag bilden, ein elastisches Element zur Anschlagsdämpfung aufweist. Bevorzugt ist ein solches elastisches Element ein auf den an sich steifen Werkstoff des Gliedes aufgebrachter elastischer Werkstoff, insbesondere ein elastisches Polymer in der Art eines in die Schulterwand auf- oder eingesetzten Elements oder in der Art einer Gummierungsschicht. In einer alternativen Ausgestaltung ist dort durch gezielte Materialaussparung oder eine eingefräste Nut in dem eigentlich steifen Werkstoff des Glieds ein elastisches, das heißt federnd nachgiebiges Funktionselement gebildet.

Das Stützband dieser Erfindung ist aus mehreren gleichartigen Gliedern aufgebaut. Die Erfindung erlaubt so vorteilhafterweise, dass einzelne Glieder der Kette entfernt oder hinzugefügt werden können, um die Länge des Stützelements oder der Koppelbrücke an die anatomischen Verhältnisse und/oder das jeweilige Therapieziel anzupassen. Durch einen speziell gestalteten Rastmechanismus der Verkopplung können die Glieder von einem Orthopädietechniker reversibel voneinander getrennt oder neu zusammengesteckt werden.

Die Gliederkette als Bestandteil des erfindungsgemäßen Stützbands weist am jeweiligen Ende bevorzugt anders gestaltete Endglieder auf, welche spezifisch ausgebildet sind, um die Glieder der Gliederkette, und damit das Stützband als solches, mechanisch mit den übrigen Elementen der Orthese, insbesondere anderen Hartrahmenabschnitten, Gelenkschienen oder Stützrahmen, zu verbinden. Bei einer Hartrahmenorthese mit gelenkübergreifendem Hartrahmensystem dient das Endglied der Gliederkette jeweils zur Verkopplung der Gliederkette mit einer zum Gelenk verlaufenden Gelenkschiene, und insbesondere zur jeweiligen Verkopplung von zwei beiderseits des Gelenks verlaufenden Gelenkschienen. In dieser spezifischen Ausgestaltung dient das erfindungsgemäße Stützband funktional als mechanisch feste Koppelbrücke zwischen den beiden seitlichen Gelenkschienen. Vorteilhafterweise erlaubt das flexible Stützband dieser Erfindung eine anatomisch gerechte und auch anpassbare Fassung des distalen und/oder proximalen Abschnitts der Extremität. Das Stützband zeigt eine Flexibilität und Beweglichkeit und kann der anatomischen Kontur der Extremität auch in der Bewegung gut folgen und liegt so immer gut an der Extremität an. Außerdem erlaubt die so gebildete Koppelbrücke zwischen den beiden seitlichen Gelenkschienen, dass diese sich gegeneinander parallel verschieben können, was eine dynamische Positionierung der Gelenkschiene über dem Körpergelenk in der Bewegung ermöglicht und die Stützfunktion in jeder Bewegungsphase verbessert; die Positionierung ist daher weitgehend selbstfindend. Eine unerwünschte Migration der Orthese kann so verhindert werden.

In einer bevorzugten Variante ist das Endglied der Gliederkette über einen oder mehrere Exzenterelemente mit der Gelenkschiene verbunden, beispielsweise über Exzenterschrauben. Dadurch ist eine zusätzliche Verstellung der Winkel von Endglied - und damit des Stützbandes - zu der Gelenkschiene ermöglicht. Eine verbesserte anatomische Anformung von Gelenkschiene und Hartrahmen kann so erreicht werden. Das ist vor allem von Vorteil, wenn in der Gelenkschiene ein multiaxiales oder selbstfindendes Gelenk eingesetzt ist.

Vor allem in der Ausgestaltung als extremitätenumgreifende (Koppel-)Brücke in einer Hartrahmenorthese ist bevorzugt ein symmetrischer Aufbau der Gliederkette vorgesehen. Dazu weist die Gliederkette bevorzugt ein bevorzugt mittig positioniertes besonders gestaltetes Mittelglied auf, das einen im Wesentlichen spiegelsymmetrischen Aufbau hat. Besonders sind an dem Mittelglied beiderseits entsprechend übergreifende Gliederabschnitte ausgebildet, um mit den entsprechenden untergreifenden Gliederabschnitten der beiderseits daran verkoppelbaren Glieder zu überlappen, wodurch die Ausrichtung der Kettenglieder innerhalb der Gliederkette an diesem Mittelglied umgekehrt wird.

Durch die erfindungsgemäße einachsige Verkopplung der Glieder untereinander, welche eine Verschwenkung der Glieder innerhalb der Primärebene des flachen Stützbands zu dessen Längsachse ermöglicht, ist in bevorzugten Ausgestaltungen zusätzlich vorgesehen, dass die Glieder zueinander in der Längsachse des Stützbands auch senkrecht zur primären Ebene des flachen Stützbandes biegbar sind, um das flache Stützband in einem Bogen zu führen. Dazu ist in einer ersten Ausgestaltung bevorzugt vorgesehen, dass die Glieder jeweils eine gewisse Eigenelastizität aufweisen. Dies kann durch Wahl des Werkstoffes des Gliedes, durch lokale Materialverdünnungen, aber auch durch Eigenelastizität allein des zum Verkoppeln der Glieder vorgesehenen Lagerzapfens verwirklicht sein. In einer alternativen oder zusätzlichen Variante ist die Lagerbuchse des jeweils benachbarten Gliedes derart dimensioniert und geformt, dass ein Lagerzapfen dort nicht spielfrei geführt ist, sondern in der Lagerbuchse kippen kann (Kippspiel). So kann eine Kippbewegung der benachbarten Glieder zueinander in gewissen Grenzen ermöglicht werden. Dadurch wird es vorteilhafterweise möglich, das an sich flache Stützband in eine Bogenform zu bringen, insbesondere um dieses an eine Extremitätenkontur anzupassen. In anderen Ausgestaltungen des erfindungsgemäßen Stützbands sind die Glieder diesbezüglich kippfest verkoppelt, um hier eine Stützwirkung zu vermitteln. Dies ist insbesondere für die Anwendung in Wirbelsäulen- oder Rückenorthesen, wenn beispielsweise einer Kyphose oder Lordose stützend entgegengewirkt werden soll, das erfindungsgemäße Stützband aber eine Seitwärtsbewegung der Wirbelsäule oder bei Skoliose erlauben soll. In einer weiteren Ausgestaltung sind in dem Stützband entlang der Gliederkette Abschnitte spielfreier, kippfester Verkopplung und Abschnitte mit Kippspiel und/oder flexiblen Gliedern ausgebildet, um die Stützwirkung an die anatomischen Verhältnisse und/oder das Therapieziel anpassbar zu machen. In dieser Variante werden Glieder mit spielfreien Verkopplungen und andere Glieder mit Verkopplungen mit Kippspiel und/oder flexible Glieder bereitgestellt, die jeweils in der Art eines Baukastens zusammen gesteckt werden können, um diese Abschnitte an der Gliederkette zu bilden. Durch einen speziell gestalteten Rastmechanismus der Verkopplung können die verschiedenen Glieder von einem Orthopädietechniker reversibel voneinander getrennt und zusammengesteckt oder ausgetauscht werden.

Gegenstand der Erfindung ist auch eine Hartrahmen-Gelenkorthese, welche insbesondere zwei gegenüberliegende Gelenkschienen aufweist, welche entlang des Körpergelenks verlaufen, wobei die Gelenkschienen über das flexible Stützband dieser Erfindung proximal und/oder distal des Gelenks miteinander verkoppelt sind. In einer besonderen Ausgestaltung davon sind die Endglieder derart ausgestaltet, dass darin die Gelenkschienen jeweils verschiebbar geführt sind. Die Endglieder dienen in dieser Ausgestaltung als Aufnahmeelement der Gelenkschienen. Sie dienen bevorzugt weiter als Anlenkpunkt von Gurten zur Fassung der jeweiligen Extremitätenabschnitte, beispielsweise Oberschenkelfassung und/oder Unterschenkelfassung. Derartige Gelenkorthesen sind Kniegelenksorthesen, Ellenbogenorthesen, Handgelenksorthesen, Fingergelenksorthesen, Fußgelenk-, Knöchelorthesen, Zehengrundgelenksorthesen und dergleichen.

Schließlich ist ein weiterer Gegenstand der Erfindung eine Rückenorthese, spezifisch zur Stützung der Wirbelsäule, worin das flexible Stützband dieser Erfindung als Stützelement zur Stützung der Wirbelsäule enthalten ist.

Die Erfindung wird durch die nachfolgenden Ausführungsbeispiele näher erläutert, ohne dass diese beschränkend zu verstehen sind.

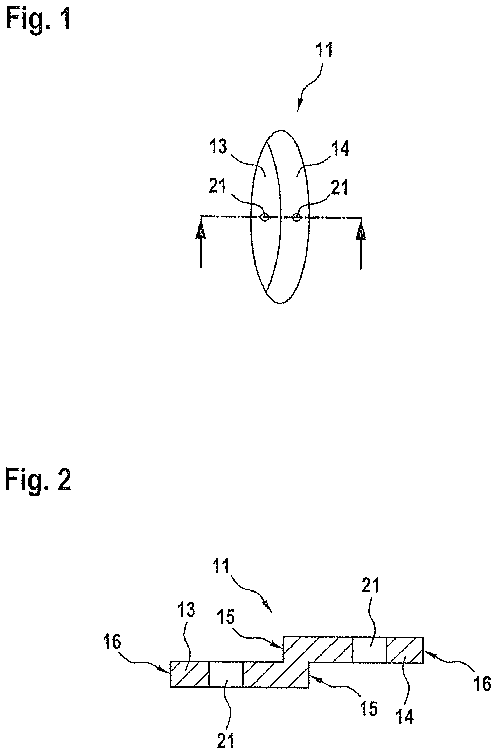

Figur 1 zeigt auf ein einzelnes Glied 11 der erfindungsgemäßen Gliederkette. Figur 2 zeigt eine Schnittansicht an der in Figur 1 angedeuteten Schnittlinie: Das Glied 11 besitzt einen flachen Grundkörper. Die hier dargestellte Ausführung weißt beiderseits außermittige Ausnehmungen 21 auf, die als Lagerbuchsen zur Aufnahme von Lagerbolzen oder -zapfen 20 zur Verkopplung mehrerer Glieder miteinander ausgebildet sind. Jedes Glied 11 weist einen untergreifenden Abschnitt 13 und einen ein benachbartes Glied übergreifenden Abschnitt 14 auf. An dem Glied 11 sind jeweils Schulterflächen oder -kanten 15, 16 ausgebildet, die bei Verkopplung der Glieder miteinander und Verschwenkung der Glieder in einer durch Lagerbuchse 21 und Lagerbolzen 20 gebildeten Achse als Anschlag dieser Verschwenkung dienen.

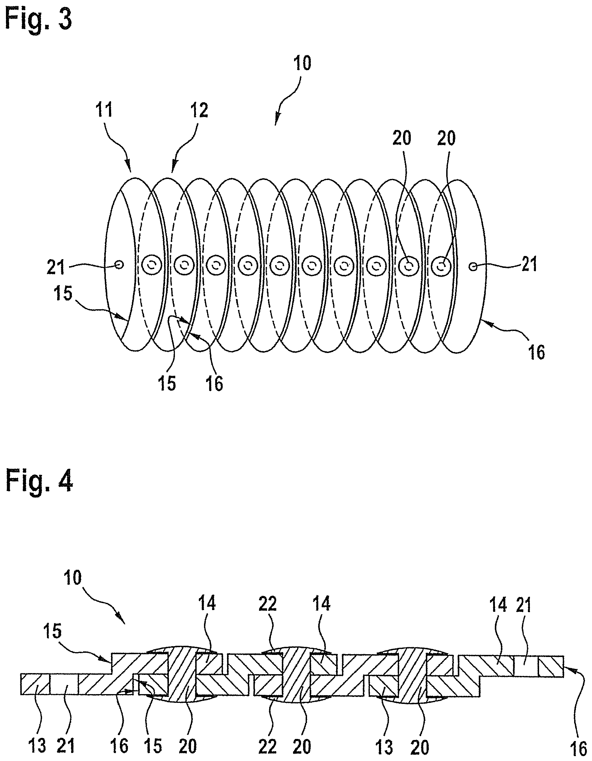

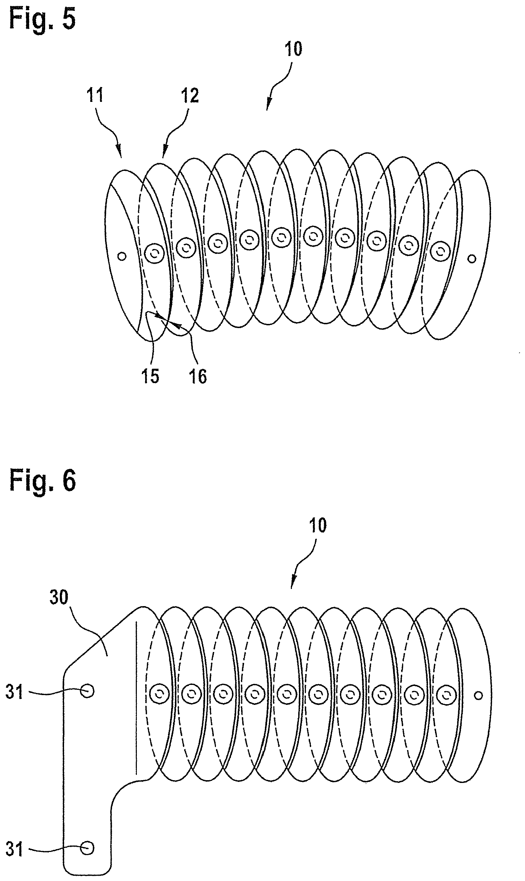

Figur 3 zeigt die Draufsicht auf einen Abschnitt einer Gliederkette 10 mit erfindungsgemäß miteinander verkoppelten Glieder 11, 12. Figur 4 zeigt eine Schnittansicht davon an der in Figur 3 angedeuteten Schnittlinie: Ein erstes Glied 11 ist dabei jeweils mit einem dazu benachbarten Glied 12 verkoppelt. Bei Verkopplung der Glieder 11,12 überlappen sich die über- und untergreifenden Abschnitte 13,14 der jeweiligen Glieder, so dass ein übergreifender Abschnitt 14 eines Glieds 14 flächig mit einem untergreifenden Abschnitt 13 des benachbarten Glieds 12 in Kontakt steht. Die Verkopplung erfolgt durch einen separaten Lagerzapfen 20, der hier durch jeweils zwei Lagerbuchsen 21 benachbarter Glieder 11,12 geführt ist und diese so verkoppelt. Der Lagerzapfen 20 ist hier als Niet mit beiderseitigen Nietköpfen 22 ausgebildet. Die an den Abschnitten und Außenkanten der jeweiligen Glieder 11,12 gebildeten Schultern 15, 16 stehen bei Verschwenkung der Glieder zueinander in körperlichem Kontakt. Figur 5 zeigt die Gliederkette 10 der Figur 3 bei Verschwenkung der Glieder gegeneinander: Die Schultern 16 und 15 benachbarter Glieder stoßen dabei jeweils aneinander, um die Verschwenkung zu limitieren.
Figur 6 zeigt die Draufsicht auf einen Endabschnitt der erfindungsgemäßen Gliederkette 10 mit einem speziellen Endglied 30 zur mechanischen Verkopplung der Gliederkette mit den übrigen Bestandteilen einer Orthese, welches geeignete Mittel 31, hier: Ausnehmungen zur Aufnahme von Schrauben oder Nieten. Am Endglied 30 ist ebenfalls ein Lagerzapfen 20 oder eine Lagerbuchse 21 ausgebildet zum Zwecke der Verkopplung mit dem ersten Glied 11 der weiteren Gliederkette aus gleichartigen Gliedern. Das Endglied weist in der dargestellten Ausführung einen übergreifenden Abschnitt 34 auf, der mit einem untergreifenden Abschnitt 13 des ersten Glieds 11 bei Verkopplung überlappt.

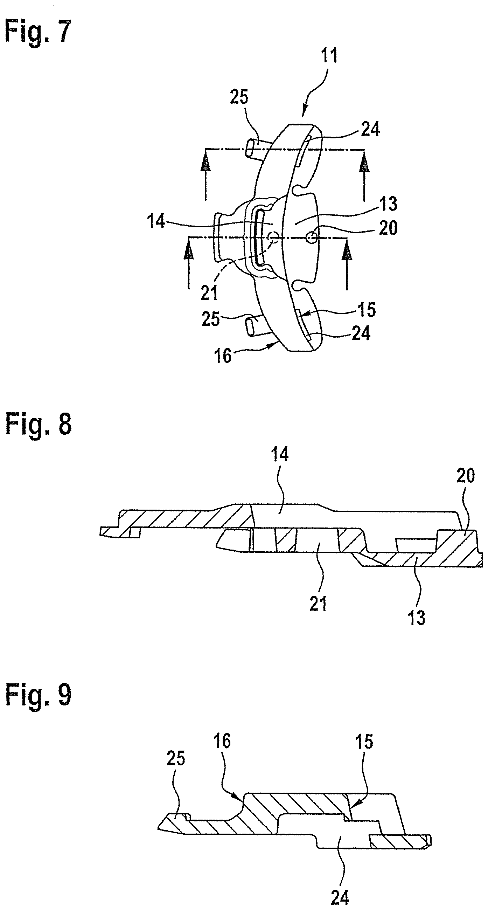

Figur 7 zeigt eine weitere Ausführung eines einzelnen Kettenglieds 11 einer erfindungsgemäßen Gliederkette. Figuren 8 und 9 zeigen jeweils Schnittansichten dieses Glieds an den in Figur 7 angedeuteten Schnittlinien: Figur 8 zeigt den zentralen Schnitt, Figur 9 den Schnitt im Bereich der Rastnase 25. Jedes Glied 11 besitzt zumindest einen übergreifenden Abschnitt 14 und zumindest einen untergreifenden Abschnitt 13. An dem untergreifenden Abschnitt 13 ist in der dargestellten Ausführung ein integrierter Lagerzapfen 20 vorgesehen, der eine Lagerbuchse 21 eines benachbarten Glieds eingreifen kann, um die Glieder zu verkoppeln. Der Lagerzapfen 20 ist mit dem Glied 11 einstückig ausgebildet. In der dargestellten Ausführung weist jedes Glied zusätzliche Rastnasen 25 auf, die in Ausnehmungen 24 eines benachbarten Glieds eingreifen können, um ein Abheben der Glieder voneinander aus der Lagerbuchse 21 zu verhindern und gleichzeitig gegebenenfalls eine unerwünschte übermäßige Torsion der Gliederkette in ihrer Längsachse verhindern.

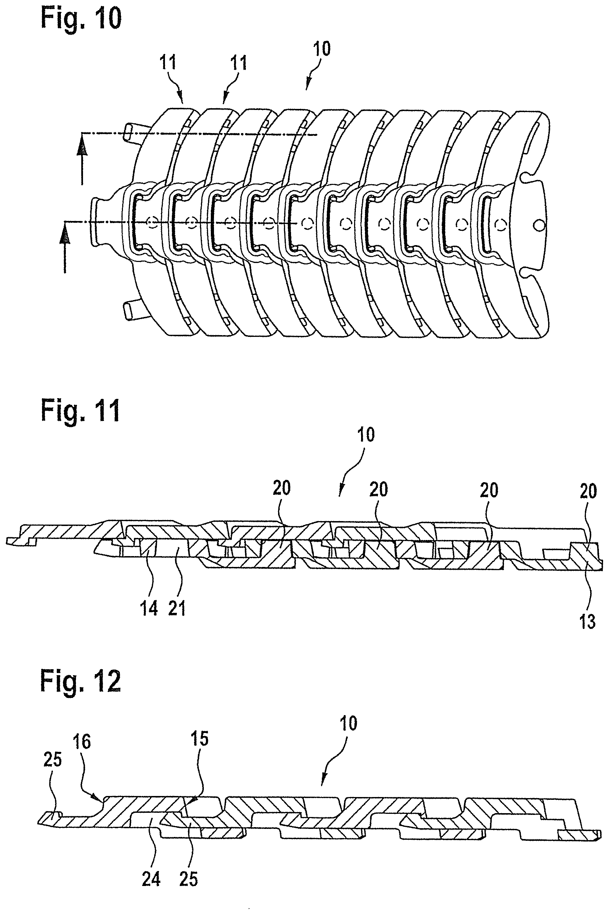

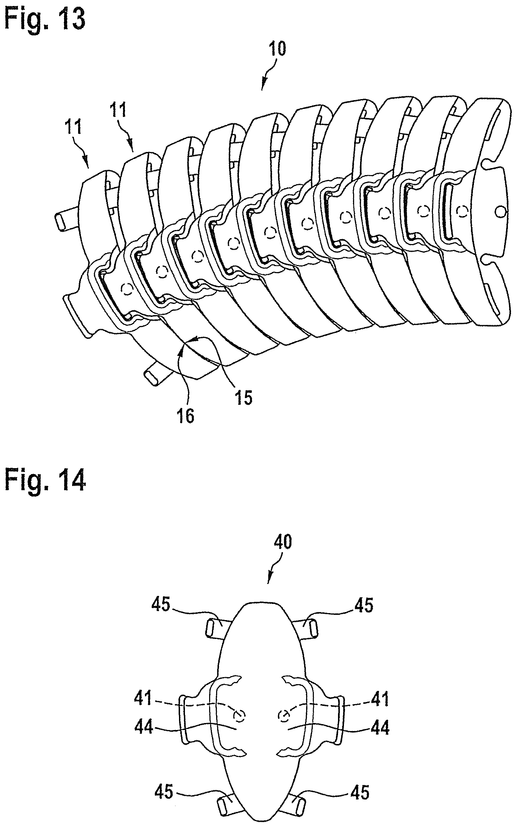

Figur 10 zeigt die Draufsicht auf einen Abschnitt einer Gliederkette 10 mit erfindungsgemäß miteinander verkoppelten Glieder 11, 12. Figuren 11 und 12 zeigen jeweils Schnittansichten dieses Glieds an den in Figur 7 angedeuteten Schnittlinien: Figur 11 zeigt den zentralen Schnitt, Figur 12 den Schnitt im Bereich der Rastnasen 25. Ein erstes Glied 11 ist dabei jeweils mit einem dazu benachbarten Glied 12 verkoppelt. Bei Verkopplung der Glieder 11,12 überlappen sich die über- und untergreifenden Abschnitte 13,14 der jeweiligen Glieder, Die an den Abschnitten und Außenkanten der jeweiligen Glieder 11,12 gebildeten Schultern 15, 16 stehen bei Verschwenkung der Glieder zueinander in körperlichem Kontakt. Figur 13 zeigt die Gliederkette 10 der Figur 10 bei Verschwenkung der Glieder gegeneinander: Die Schultern 16 und 15 benachbarter Glieder stoßen dabei jeweils aneinander, um die Verschwenkung zu limitieren.

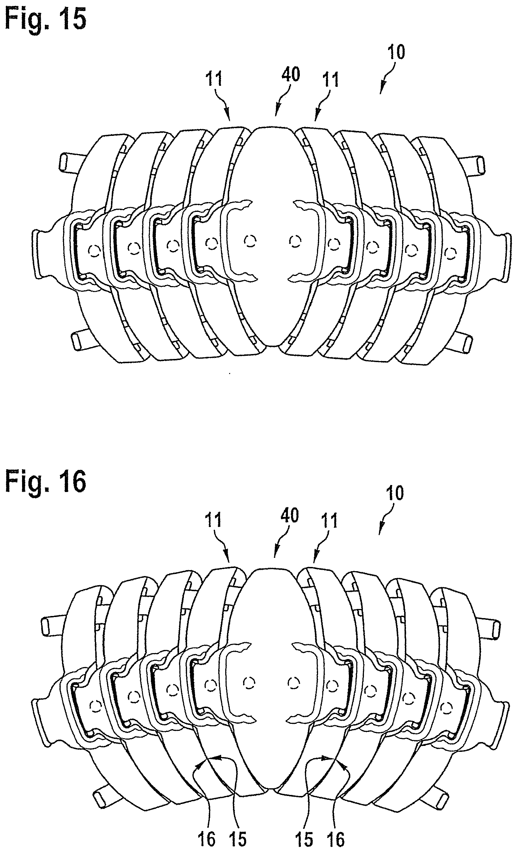

Figur 14 zeigt ein spezielles Mittelglied 40 der erfindungsgemäßen Gliederkette 10, welches beiderseits übergreifende Abschnitte 44 analog zu den übergreifenden Abschnitten 14 eines einzelnen gleichartigen Gliedes 11 sowie Lagerbuchsen 41, gleichartig zu den Lagerbuchsen 21 des einzelnen gleichartigen Gliedes 11 aufweist. Das Mittelstück 40 dient zur "Symmetrierung" der Ausrichtung der gleichartigen Glieder 11 in der Kette beiderseits des Mittelstücks 40. In der dargestellten Ausführung sind zusätzlich Rastnasen 45 ausgebildet, die in entsprechende Ausnehmungen 24 der beiderseits verkoppelbaren gleichartigen Glieder 11 eingreifen können. Die Figuren 15 und 16 zeigen jeweils Draufsichten auf einen Ausschnitt aus einer Ausführung der erfindungsgemäßen Gliederkette 10, welche aus gleichartigen Gliedern 11 und einem symmetrierenden Mitelstück 40 aufgebaut sind. Figur 15 zeigt diese Gliederkette 10 in gestreckter Anordnung, Figur 16 zeigt die Gliederkette bei Verschwenkung der einzelnen Glieder jeweils in den durch Lagerzapfen 20 und Lagerbuchse 21 gebildeten Verschwenkachsen bis zum Endanschlag der Verschwenkung an den jeweiligen Schultern 16, 15 der Glieder.

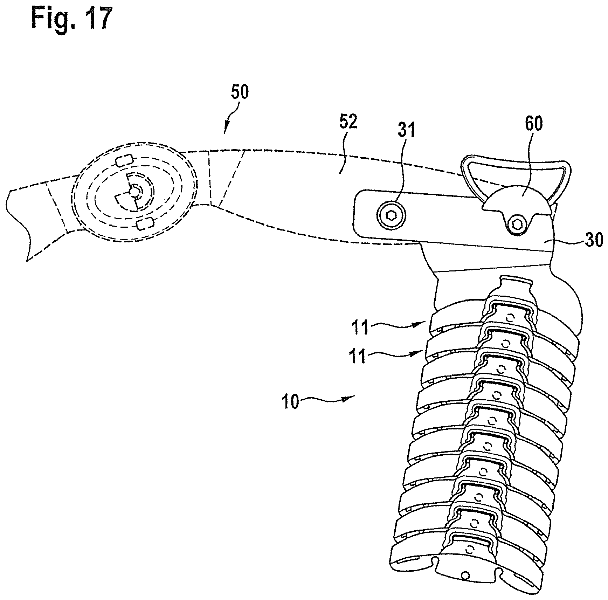

Figur 17 ist eine schematische Darstellung eines Ausschnittes einer Hartrahmen-Kniegelenkorthese mit einem Abschnitt des erfindungsgemäßen Stützbands aus Gliederkette 10 mit gleichartigen Gliedern 11 und Endglied 30. In dieser Ausführung ist die Gelenkschienen 50 mit einem Gelenkschenkel 52 an dem spezifisch ausgebildeten Endglied 30 in Ausnehmungen 31 verschraubt. Zusätzlich sind an dem Endglied gegebenenfalls abrüstbare Laschen 60 zur Aufnahme und Fixierung von Gurtbändern ausgebildet.

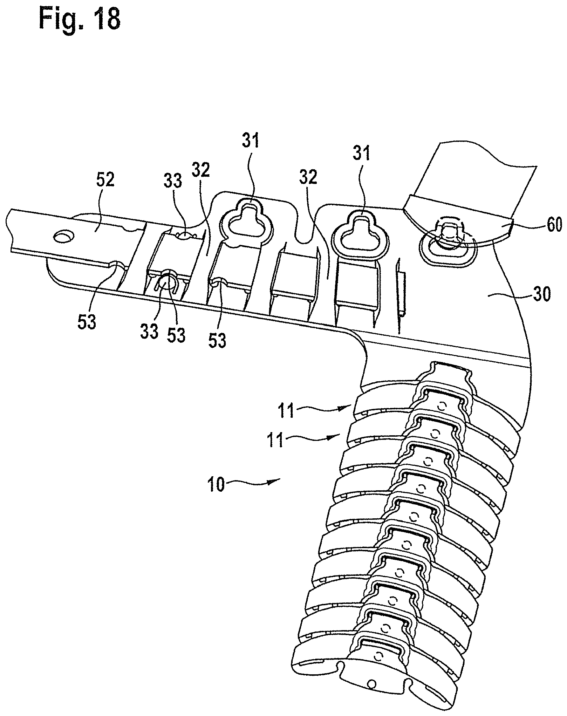

Figur 18 ist eine schematische Darstellung eines Ausschnittes einer Hartrahmen-Kniegelenkorthese mit einem Abschnitt des erfindungsgemäßen Stützbands aus Gliederkette 10 mit gleichartigen Gliedern 11 und Endglied 30. In dieser Ausführung ist der Gelenkschenkel 52 der Gelenkschiene der Orthese in dem spezifisch ausgebildeten Endglied 30 in Führungslaschen 32 geführt und formschlüssig gehalten. Zur Verstellung der effektiven Länge der Gelenkschiene ist der Gelenkschenkel 52 rastend in dem Endglied verschiebbar und an Rastelementen 33 des Endglieds 30, die in Rastkerben 53 des Gelenkschenkels 52 eingreifen können, festlegbar. Zusätzlich sind an dem Endglied gegebenenfalls eine oder mehrere abrüstbare Laschen 60 zur Aufnahme und Fixierung von Gurtbändern ausgebildet. In der dargestellten Ausführung rasten die Laschen 60 in spezifisch geformte Ausnehmungen 31 an dem Endglied 30 des Stützelements ein.

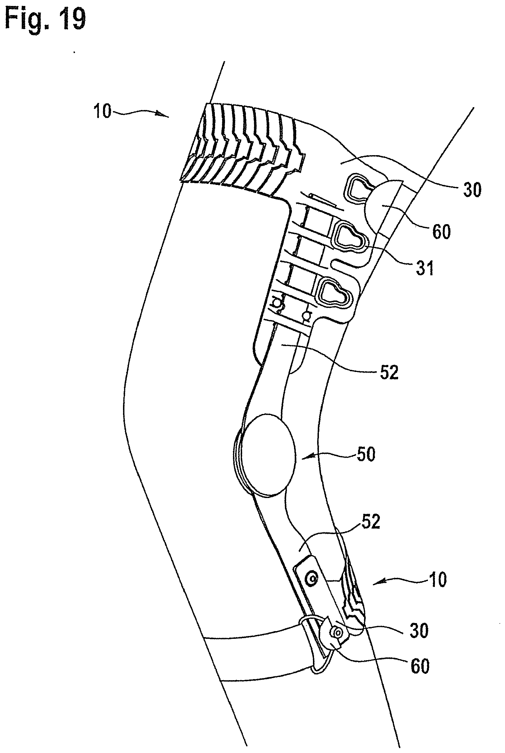

Figur 19 ist eine schematische Perspektivansicht einer Ausführung einer erfindungsgemäßen Hartrahmen-Gelenkorthese, welche die erfindungsgemäßen flexiblen Stützbänder als Koppelbrücken zwischen den beiden Gelenkschienen aufweist.

## Patentansprüche

1. Flexibles Stützband für eine Orthese, enthaltend eine flache Gliederkette (10) mit mehreren gleichartigen flachen Gliedern (11,12), wobei jeweils ein erstes Glied (11) mit dem benachbarten zweiten Glied (12) in jeweils untergreifenden und übergreifenden Gliederabschnitten (13,14) körperlich überlappend über einen dazu senkrechten Lagerzapfen (20) zugfest verkoppelt ist und die Glieder (11,12) um die Achse des Lagerzapfens (20) gegeneinander verschwenkbar sind, wobei die Schultern (15,16) überlappender Gliederabschnitte (13,14) einen Endanschlag zur Verschwenkungslimitierung bilden, **dadurch gekennzeichnet, dass** das erste Glied (11) jeweils mindestens ein Rastelement (25) aufweist, das in eine Ausnehmung (24) des benachbarten Glieds (12) eingreift, um ein Abheben der Glieder (11,12) voneinander zu blockieren.

2. Stützband nach Anspruch 1, wobei der Lagerzapfen (20) an dem untergreifenden einen Gliederabschnitt (13) des ersten Glieds (11) ausgebildet ist und eine Lagerbuchse (21) an dem übergreifenden anderen Gliederabschnitt (14) des benachbarten zweiten Glieds (12) ausgebildet ist und der Lagerzapfen (20) in die Lagerbuchse (21) eingreift, um die Glieder (11,12) zu verkoppeln.

3. Stützband nach Anspruch 1 oder 2, wobei zumindest eine der Schultern (15,16) ein elastisches Element zur Anschlagsdämpfung aufweist.

4. Stützband nach einem der vorstehenden Ansprüche, wobei die Gliederkette (10) jeweils ein Endglied (30) aufweist, worüber die Gliederkette an der Orthese fixierbar ist.

5. Stützband nach einem der vorstehenden Ansprüche, wobei die Gliederkette (10) ein zentrales Mittelglied (40) enthält, das einen spiegelsymmetrischen Aufbau beiderseits mit übergreifenden Gliederabschnitten (44) aufweist.

6. Stützband nach einem der vorstehenden Ansprüche, wobei der Lagerzapfen (20) zusätzlich einen gegenüber dem Durchmesser der Lagerbuchse (21) verdickten Kopf (21) aufweist, um ein Abheben der Glieder (11,12) voneinander zu blockieren.

7. Hartrahmen-Gelenkorthese, enthaltend das flexible Stützband nach einem der vorstehenden Ansprüche und zwei gegenüberliegende Gelenkschienen (50), wobei die beiden Gelenkschienen (50) über das flexible Stützband nach einem der vorstehenden Ansprüche mit einander mechanisch verkoppelt sind.

8. Hartrahmen-Gelenkorthese nach Anspruch 7, wobei ein Schenkel (52) der Gelenkschiene (50) in einem Endglied (30) des Stützbands jeweils verschiebbar geführt ist.

9. Rückenorthese, enthaltend das flexible Stützband nach einem der Ansprüche 1 bis 6 zur Stützung der Wirbelsäule.

## Claims

1. Flexible support band for an orthosis, containing a planar link chain (10) having a plurality of similar planar links (11, 12), in each case a first link (11) being firmly coupled to the adjacent second link (12), in link portions (13, 14) that engage from below and from above respectively, so as physically to overlap above a bearing journal (20) perpendicular thereto, and the members (11, 12) being pivotable with respect to one another about the axis of the bearing journal (20), the shoulders (15, 16) of overlapping link portions (13, 14) forming an end stop for limiting the pivoting, **characterised in that** the first member (11) in each case has at least one latch element (25), which engages in a recess (24) of the adjacent member (12) so as to block the links (11, 12) from lifting away from one another.

2. Support band according to claim 1, wherein the bearing journal (20) is formed on one link portion (13), which engages from below, of the first member (11), and a bearing bushing (21) is formed on the other link portion (14), which engages from above, of the adjacent second member (12), and the bearing journal (20) engages in the bearing bushing (21) so as to couple the links (11, 12).

3. Support band according to either claim 1 or claim 2, wherein at least one of the shoulders (15, 16) has an elastic element for stop damping.

4. Support band according to any of the preceding claims, wherein the link chain (10) in each case has an end member (30) via which the link chain can be fixed to the orthosis.

5. Support band according to any of the preceding claims, wherein the link chain (10) includes a central intermediate link (40) which has a mirror-symmetrical construction on both sides with link portions (44) which engage from above.

6. Support band according to any of the preceding claims, wherein the bearing journal (20) additionally has a head (21) which is thickened with respect to the diameter of the bearing bushing (21), so as to block the links (11, 12) from lifting away from one another.

7. Rigid-frame joint orthosis, containing the flexible support band according to any of the preceding claims and two opposite articulated rails (50), wherein the two articulated rails (50) are mechanically coupled to one another via the flexible support band according to any of the preceding claims.

8. Rigid-frame joint orthosis according to claim 7, wherein a limb (52) of the articulated rail (50) is guided displaceably in an end member (30) of the support band in each case.

9. Back orthosis, containing the flexible support band according to any of claims 1 to 6 for supporting the spine.

## Revendications

1. Bande de support flexible pour une orthèse, contenant une chaîne à maillons plate (10) dotée de plus maillons plats identiques (11, 12), dans laquelle un premier maillon (11) est respectivement couplé de manière inextensible au deuxième maillon adjacent (12) se recouvrant physiquement dans des sections de maillon (13, 14) respectivement au-dessus et en dessous et par le biais d'un tourillon vertical (20) et les maillons peuvent pivoter l'un par rapport à l'autre autour de l'axe du tourillon (20), dans laquelle les sections de maillon (13, 14) recouvrant les épaules (15, 16) forment une butée de fin destinée à limiter le pivotement, **caractérisée en ce que** le premier maillon (11) comporte respectivement au moins un élément de cliquet (25), lequel entre en prise dans un évidement (24) du maillon adjacent (12), afin de bloquer un décollement des maillons (11, 12) l'un par rapport à l'autre.

2. Bande de support selon la revendication 1, dans laquelle le tourillon (20) est réalisé sur l'une section de maillon (13) de dessous du premier maillon (11) et une douille de tourillon (21) est réalisée sur l'autre section de maillon (14) de dessus du deuxième maillon (12) et le tourillon (20) entre en prise dans la douille de tourillon (21), afin de coupler les maillons (11, 12).

3. Bande de support selon la revendication 1 ou 2, dans laquelle au moins une des épaules (15, 16) comporte un élément élastique pour amortir la butée.

4. Bande de support selon l'une des revendications précédentes, dans laquelle la chaîne de maillons (10) comporte respectivement un maillon terminal (30), par le biais duquel la chaîne de maillons peut être fixée à l'orthèse.

5. Bande de support selon l'une des revendications précédentes, dans laquelle la chaîne de maillons (10) contient un maillon intermédiaire central (40), lequel comporte une structure symétrique en miroir dotée des deux côtés de sections de maillons (44) de dessus.

6. Bande de support selon l'une des revendications précédentes, dans laquelle le tourillon (20) comporte en outre une tête (21) épaissie par rapport au diamètre de la douille de tourillon (21), afin de bloquer un décollement des maillons (11, 12) l'un par rapport à l'autre.

7. Orthèse articulée à cadre rigide, contenant la bande de support flexible selon l'une des revendications précédentes et deux rails d'articulations (50) se faisant face, dans laquelle les deux rails d'articulations (50) sont mécaniquement couplés l'un à l'autre par le biais de la bande de support flexible selon l'une des revendications précédentes.

8. Orthèse articulée à cadre rigide selon la revendication 7, dans laquelle une patte (52) du rail d'articulation (50) est respectivement guidée en coulissement dans un maillon terminal (30) de la bande de support flexible.

9. Orthèse dorsale contenant la bande de support flexible selon l'une des revendications 1 à 6 pour soutenir la colonne vertébrale.
